# EUROPÄISCHE PATENTSCHRIFT

(11) **EP 0 781 317 B1**
(45) Veröffentlichungstag und Bekanntmachung des Hinweises auf die Patenterteilung: **03.02.1999**
(21) Anmeldenummer: 95931961.7
(22) Anmeldetag: 01.09.1995
(51) Int. Cl.: C11D 1/37, B01F 17/00

(54) **MILDE DETERGENSGEMISCHE**
MILD DETERGENT MIXTURES
MELANGES DETERGENTS DOUX

(30) Priorität: 12.09.1994 DE 4432366
(43) Veröffentlichungstag der Anmeldung: 02.07.1997
(73) Patentinhaber: Henkel Kommanditgesellschaft auf Aktien, 40191 Düsseldorf (DE)
(72) Erfinder: FABRY, Bernd, D-41352 Korschenbroich (DE); BEHLER, Ansgar, D-46240 Bottrop (DE)
(86) Internationale Anmeldenummer: EP9503436
(87) Internationale Veröffentlichungsnummer: WO9608549

(56) Entgegenhaltungen:
- EP-A- 0 256 656
- EP-A- 0 318 729
- EP-A- 0 559 375
- WO-A-95/23582
- BE-A- 622 463
- DE-A- 4 300 325
- FR-A- 1 208 750
- FR-A- 1 272 254
- GB-A- 941 807
- GB-A- 954 046

## Beschreibung

### Gebiet der Erfindung

Die Erfindung betrifft die Verwendung von Detergensgemischen mit verbesserter hautkosmetischer Verträglichkeit, enthaltend Monoglycerid(ether)sulfate und ausgewählte Fettsäurekondensationsprodukte, zur Herstellung von oberflächenaktiven Mitteln.

### Stand der Technik

Monoglyceridsulfate stellen formal Anlagerungsprodukte von Schwefeltrioxid an die primäre Hydroxylgruppe eines Glycerinmonofettsäureesters dar. Technisch gesehen handelt es sich jedoch um komplexe Aniontensidgemische, die üblicherweise durch gleichzeitige Umesterung und Sulfatierung von Gemischen aus Trigylceriden und Glycerin sowie nachfolgender Neutralisation erhalten werden.

Monoglyceridsulfate zeichnen sich durch zufriedenstellende anwendungstechnische Eigenschaften und gute dermatologische Verträglichkeit aus. Übersichten zu Herstellung und Eigenschaften von Monoglyceridsulfaten sind beispielsweise von A.K.Biswas et al. in **J.Am.Oil.Chem.Soc. 37, 171 (1960)**, R.Chamanial et al. in **J.Oil.Technol.Ass.Ind. 41 (1972)** und J.K.Jain in **Indian J.Pharm.Sci. 41, 181 (1979)** erschienen.

Für eine Reihe von Anwendungen ist das Schaumvermögen, insbesondere bei Härtebelastung, sowie die hautkosmetische Verträglichkeit der Monoglyceridsulfate sowie der dazu analogen Ethersulfate nicht voll befriedigend.

Die Aufgabe der Erfindung hat demnach darin bestanden, einen Weg zu finden, Performance und Hautverträglichkeit von Monoglycerid(ether)sulfaten signifikant zu verbessern.

### Beschreibung der Erfindung

Gegenstand der Erfindung ist die Verwendung von milden Detergensgemischen zur Herstellung von Handgeschirrspülmitteln, Haarshampoos und Schaumbädern, dadurch gekennzeichnet, daß man, enthaltend
(a) Monoglycerid(ether)sulfate und
(b) Fettsäurekondensationsprodukte, ausgewählt aus der Gruppe, die gebildet wird von
   (b1) Fettsäureisethionaten,
   (b2) Fettsäuretauraten und/oder
   (b3) Fettsäuresarcosinaten
   im Gewichtsverhältnis der Komponenten (a) und (b) von 50:50 bis 80:20 einsetzt. Überraschenderweise wurde gefunden, daß Abmischungen von Monoglyceridsulfaten bzw. Monoglyceridethersulfaten und den genannten Fettsäurekondensationsprodukten zu einer in synergistischer Weise verbesserten hautkosmetischen Verträglichkeit bei gesteigertem Schaumvermögen führt.

### Monoglyceride und Monoglyceridethersulfate

Monoglyceridsulfate und Monoglyceridethersulfate stellen bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **WO-A-92/09569, WO-A-92/09570,** Henkel]. Die neutralisierten Stoffe können - falls gewünscht - einer Ultrafiltration unterworfen werden, um den Elektrolytgehalt auf ein gewünschtes Maß zu vermindern.

Die im Sinne der Erfindung einzusetzenden **Monoglycerid(ether)sulfate** folgen vorzugsweise der Formel (I) 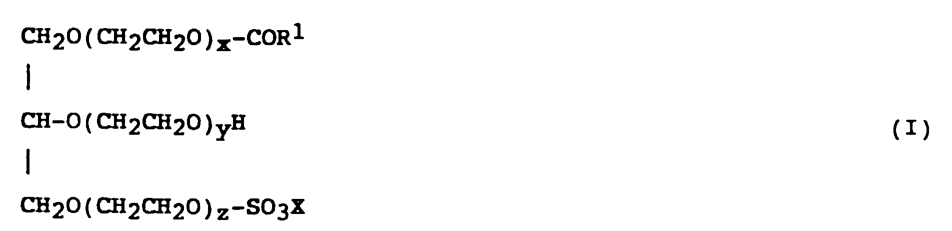 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäureonoglycerid, Stearinsäuremonoglycerid, Ölsäuremonoglycerid und Talgfettsäuremonoglycerid sowie deren Ethylenoxidaddukten mit Schwefeltrioxid oder Chlorsulfonsäure in Form ihrer Natriumsalze. Vorzugsweise werden Monoglyceridsulfate der Formel (I) eingesetzt, in der R¹CO für einen linearen Acylrest mit 8 bis 18 Kohlenstoffatomen steht.

### Fettsäureisethionate

Fettsäureisethionate stellen bekannte anionische Tenside dar, die nach den einschlägigen Methoden der präparativen organischen Chemie erhalten werden können. Üblicherweise geht man von Fettsäuren aus, die in Gegenwart alkalischer Katalysatoren mit Isethionsäure verestert werden [vgl. **US-A-4536338** (Unilever)]. Eine Übersicht zu Fettsäureisethionaten ist von R. Login in **HAPPI, Sept., 56 (1984)** erschienen.

Die Fettsäureisethionate folgen vorzugsweise der Formel **(II)**,

**R**^{**2**}**CO-CH**_{**2**}**CH**_{**2**}**SO**_{**3**}**X** (II)

in der R²CO für einen aliphatischen, linearen oder verzweigten, gesättigten oder ungesättigten Acylrest mit 6 bis 22 Kohlenstoffatomen und X für ein Alkali- und/oder Erdalkalimetall, Ammonium, Alkylammonium, Alkanolammonium oder Glucammonium steht.

Typische Beispiele stellen Fettsäureisethionate dar, die sich von der Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isosterainsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen, ableiten.

Vorzugsweise werden Fettsäureisethionate in Form ihrer Natrium- und/oder Ammoniumsalze eingesetzt, die sich von Fettsäuren mit 12 bis 18 und insbesondere 12 bis 14 Kohlenstoffatomen ableiten. Besonders bevorzugt ist der Einsatz von Kokosfettsäureisethionat-Natrium- bzw. Ammoniumsalz.

### Fettsäuretaurate

Fettsäuretaurate stellen bekannte anionische Tenside dar, die nach den einschlägigen Methoden der präparativen organischen Chemie erhalten werden können. Üblicherweise geht man von Fettsäuren aus, die in Gegenwart alkalischer Katalysatoren mit N-Methyltaurin kondensiert werden. Eine Übersicht zu diesem Thema ist von R.Bistline et al. in **J.Am.Oil.Chem.Soc. 48**, **657 (1971)** und K.Miyazawa et al. in **Cosm.Toil. 108, 81 (1993)** erschienen.

Die Fettsäuretaurate folgen vorzugsweise der Formel **(III)**, 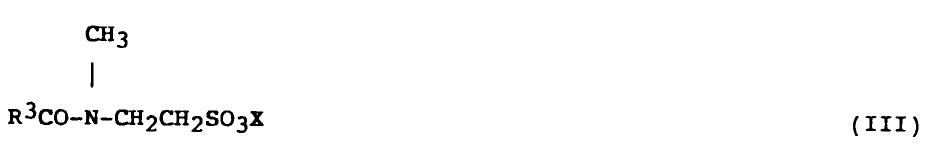 in der R³CO für einen aliphatischen, linearen oder verzweigten, gesättigten oder ungesättigten Acylrest mit 6 bis 22 Kohlenstoffatomen und X für ein Alkali- und/oder Erdalkalimetall, Ammonium, Alkylammonium, Alkanolammonium oder Glucammonium steht.

Typische Beispiele stellen Fettsäuretaurate dar, die sich von der Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isosterainsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen, ableiten.

Vorzugsweise werden Fettsäuretaurate in Form ihrer Natrium- und/oder Ammoniumsalze eingesetzt, die sich von Fettsäuren mit 12 bis 18 und insbesondere 12 bis 14 Kohlenstoffatomen ableiten. Besonders bevorzugt ist der Einsatz von Kokosfettsäuretaurat-Natrium- bzw. Ammoniumsalz.

### Fettsäuresarcosinate

Fettsäuresarcosinate stellen bekannte anionische Tenside dar, die nach den einschlägigen Methoden der präparativen organischen Chemie erhalten werden können. Üblicherweise geht man von Fettsäuren aus, die in Gegenwart alkalischer Katalysatoren mit Sarcosin (N-Methylglycin) kondensiert werden. Eine Übersicht zu Fettsäuresarcosinaten ist von A.Desai et al. in **Tens.Surf.Det. 30, 315 (1993)** erschienen.

Die Fettsäuresarcosinate folgen vorzugsweise der Formel **(IV)**, 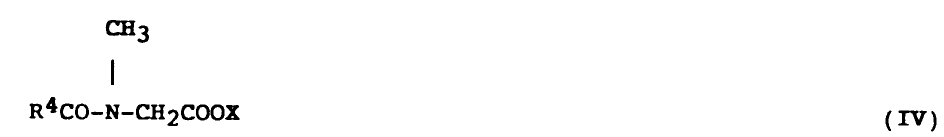 in der R⁴CO für einen aliphatischen, linearen oder verzweigten, gesättigten oder ungesättigten Acylrest mit 6 bis 22 Kohlenstoffatomen und X für ein Alkali- und/oder Erdalkalimetall, Ammonium, Alkylammonium, Alkanolammonium oder Glucammonium steht.

Typische Beispiele stellen Fettsäuresarcosinate dar, die sich von der Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isosterainsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen, ableiten.

Vorzugsweise werden Fettsäuresarcosinate in Form ihrer Natrium- und/oder Ammoniumsalze eingesetzt, die sich von Fettsäuren mit 12 bis 18 und insbesondere 12 bis 14 Kohlenstoffatomen ableiten. Besonders bevorzugt ist der Einsatz von Kokosfettsäuresarcosinat-Natrium- bzw. Ammoniumsalz.

Im Hinblick auf ein besonders niedriges Reizpotential enthalten die erfindungsgemäß verwendeten Detergensgemische die Monoglycerid(ether)sulfate und die ausgewählten Fettsäurekondensationsprodukte im Gewichtsverhältnis von 50: 50 bis 80:20.

### Tenside

Die erfindungsgemäß verwendeten Detergensgemische können weitere anionische, nichtionische, kationische und/oder amphotere Tenside enthalten.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Fettsäureamid(ether)sulfate, Mono- und Dialkylsulfosuccinate, Mono- und Dialkylsulfosuccinamate, Sulfotriglyceride, Amidseifen, Alkyloligoglucosidsulfate und Alkyl(ether)phosphate. Sofern die anionischen Tenside Polyglycoletherketten enthalten, können sie eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Mischether bzw. Mischformale, Alk(en)yloligoglykoside, Fettsäure-N-alkylglucamide und Proteinhydrolysate (insbesondere pflanzliche Produkte auf Sojabasis). Sofern die nichtionischen Tenside Polyglycoletherketten enthalten, können sie eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products",** **Springer Verlag, Berlin, 1987, S.54-124 oder J.Falbe (ed.), "Katalysatoren, Tenside und Mineralöladditive", Thieme Verlag, Stuttgart, 1978, S.123-217** verwiesen.

Die erfindungsgemäß verwendeten Detergensgemische können die oben genannten zusätzlichen Tenside in Anteilen von 1 bis 50, vorzugsweise 5 bis 25 Gew.-% - bezogen auf den Feststoffanteil der Gemische - enthalten.

### Gewerbliche Anwendbarkeit

Die erfindungsgemäß verwendeten Detergensgemische zeichnen sich durch ein besonders vorteilhaftes Schaumvermögen und eine in synergistischer Weise verbesserte hautkosmetische Verträglichkeit aus, Eigenschaften, die bei der Entwicklung einer Vielzahl von oberflächenaktiven Mitteln von Bedeutung ist:

Die vorliegende Erfindung betrifft daher die Herstellung von oberflächenaktiven Mitteln die einen Gehalt dieser Detergensgemische aufweisen und die im folgenden näher derfiniert werden:
o **Handgeschirrspülmittel**, enthaltend 10 bis 50 Gew.-% - bezogen auf das Mittel - der erfindungsgemäß verwendeten Detergensgemische sowie übliche Hilfs- und Zusatzstoffe.
o **Haarshampoos**, enthaltend 10 bis 30 Gew.-% - bezogen auf das Mittel - der erfindungsgemäß verwendeten Detergensgemische sowie übliche Hilfs- und Zusatzstoffe.
o **Schaumbäder**, enthaltend 10 bis 30 Gew.-% - bezogen auf das Mittel - der erfindungsgemäß verwendeten Detergensgemische sowie übliche Hilfs- und Zusatzstoffe.

### Hilfs- und Zusatzstoffe

**Spülmittel** auf Basis der erfindungsgemäß verwendeten Detergensgemische können - neben den bereits genannten Tensiden - als weitere Hilfs- und Zusatzstoffe beispielsweise Builder, Salze, Bleichmittel, Bleichaktivatoren, Lösungsvermittler und Enzyme enthalten.

Übliche **Builder** sind Natriumaluminiumsilicate (Zeolithe), Phosphate, Phosphonate, Ethylendiamintetraessigsäure, Nitrilotriacetat, Citronensäure und/oder Polycarboxylate.

Als Salze bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

**Haarshampoos** oder **Schaumbäder** können als weitere Hilfs- und Zusatzstoffe - neben den bereits genannten Tensiden - **Emulgatoren** wie etwa alkoxylierte Fettalkohole oder Sorbitanester enthalten.

Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid.

Unter biogenen **Wirkstoffen** sind beispielsweise Pflanzenextrakte und Vitaminkomplexe zu verstehen.

Gebräuchliche **Filmbildner** sind beispielsweise Chitosan, mikrokristallines Chitosan, quaterniertes Chitosan, Polyvinylpyrrolidon, Vinylpyrrolidon-Vinyl-acetat-Copolymerisate, Polymere der Acrylsäurereihe, quaternäre Cellulose-Derivate und ähnliche Verbindungen.

Als **Konservierungsmittel** eignen sich beispielsweise Phenoxyethanol, Formaldehydlösung, Parabene, Pentadiol oder Sorbinsäure.

Als **Perlglanzmittel** kommen beispielsweise Glycoldistearinsäureester wie Ethylenglycoldistearat, aber auch Fettsäuremonoglycolester in Betracht.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

### Beispiele

### I. Eingesetzte Tenside

Al) C_{12/18}-Kokosmonoglyceridsulfat-Natriumsalz

B1) Kokosfettsäureisethionat-Natriumsalz
B2) Kokosfettsäuretaurat-Natriumsalz
B3) Kokosfettsäuresarcosinat-Natriumsalz

### II. Anwendungstechnische Ergebnisse

Das Schaumvermögen wurde nach der DIN-Methode 53 902, Teil 2 (Ross-Miles-Test) durchgeführt. Eingesetzt wurden 1 Gew.-%ige Tensidlösungen in Wasser von 16°d; die Temperatur betrug 20°C. Bestimmt wurden Basisschaum und Schaumvolumen nach 5 min.

Die Bestimmung des Reizpotentials erfolgte gemäß der OECD-Methode No.404 und der EEC Directive 84/449 EEC, Pt.B.4. Die angegebenen Reizsummenscores wurden aus den nach 24, 48 und 72 Stunden erhaltenen Reizscores gebildet. Dabei wurde der im Vergleichsversuch V1 ermittelte Reizsummenscore für ein 100 %iges C_{12/18}-Kokosfettsäuremonoglyceridsulfat-Natriumsalz zu 100 % gesetzt und die in den übrigen Versuchen erhaltenen Reizsummenscores zu diesem ins Verhältnis gesetzt.

Die Ergebnisse sind in Tabelle 1 zusammengefaßt (Prozentangaben als Gew.-%).

**Tabelle 1**

| Schaumvermögen und Reizpotential | | | | | | |
|---|---|---|---|---|---|---|
| | | | | **Schaumhöhe [ml]** | | |
| **Bsp.** | **[A1]** | **B** | **[B]** | **sofort** | **nach 5 min** | **Reizsummen score %-rel** |
| 1 | 50 | B1 | 50 | 550 | 420 | 65 |
| 2 | 70 | B1 | 30 | 570 | 430 | 59 |
| 3 | 70 | B2 | 30 | 570 | 400 | 59 |
| 4 | 70 | B3 | 30 | 570 | 420 | 59 |
| V1 | 100 | - | - | 500 | 300 | 100 |
| V2 | 0 | B1 | 100 | 350 | 210 | 95 |
| V3 | 0 | B2 | 100 | 350 | 220 | 98 |
| V4 | 0 | B3 | 100 | 340 | 220 | 93 |

## Patentansprüche

1. Verwendung von milden Detergensgemischen zur Herstellung von Handgeschirrspülmitteln, Haarshampoos und Schaumbädern, **dadurch gekennzeichnet**, daß man
(a) Monoglycerid(ether)sulfate und
(b) Fettsäurekondensationsprodukte, ausgewählt aus der Gruppe, die gebildet wird von
(b1) Fettsäureisethionaten,
(b2) Fettsäuretauraten und/oder
(b3) Fettsäuresarcosinaten
im Gewichtsverhältnis der Komponenten (a) und (b) von 50 : 50 bis 80 : 20 einsetzt.

2. Verwendung nach Anspruch 1, **dadurch gekennzeichnet**, daß man Monoglycerid(ether)sulfate der Formel (I) einsetzt, 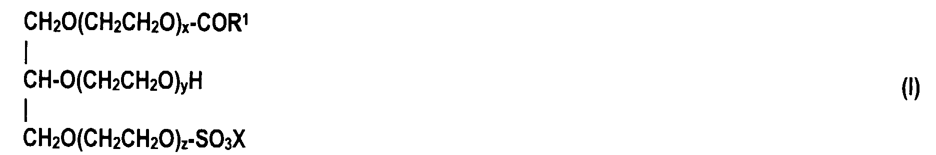 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

3. Verwendung nach den Ansprüchen 1 und 2, **dadurch gekennzeichnet**, daß man Fettsäureisethionate der Formel **(II)** einsetzt,
**R**^{**2**}**CO-CH**_{**2**}**CH**_{**2**}**SO**_{**3**}**X** (II)
in der R²CO für einen aliphatischen, linearen oder verzweigten, gesättigten oder ungesättigten Acylrest mit 6 bis 22 Kohlenstoffatomen und X für ein Alkali- und/oder Erdalkalimetall, Ammonium, Alkylammonium, Alkanolammonium oder Glucammonium steht.

4. Verwendung nach den Ansprüchen 1 bis 3, **dadurch gekennzeichnet**, daß man Fettsäuretaurate der Formel **(III)** einsetzt, 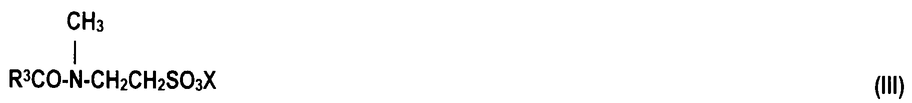 in der R³CO für einen aliphatischen, linearen oder verzweigten oder ungesättigten Acylrest mit 6 bis 22 Kohlenstoffatomen und X für ein Alkali- und/oder Erdalkalimetall, Ammonium, Alkylammonium, Alkanolammonium oder Glucammonium steht.

5. Verwendung nach den Ansprüchen 1 bis 4, **dadurch gekennzeichnet**, daß man Fettsäuresarcosinate der Formel **(IV)** einsetzt, 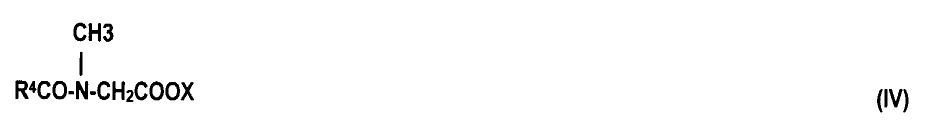 in der R⁴CO für einen aliphatischen, linearen oder verzweigten oder ungesättigten Acylrest mit 6 bis 22 Kohlenstoffatomen und X für ein Alkali- und/oder Erdalkalimetall, Ammonium, Alkylammonium, Alkanolammonium oder Glucammonium steht.

## Claims

1. The use of mild detergent mixtures for the production of manual dishwashing detergents, hair shampoos and foam baths, characterized in that
(a) monoglyceride (ether) sulfates and
(b) fatty acid condensation products selected from the group consisting of
(b1) fatty acid isethionates,
(b2) fatty acid taurates and/or
(b3) fatty acid sarcosinates
are used in a ratio by weight of component (a) to component (b) of 50:50 to 80:20

2. The use claimed in claim 1, characterized in that monoglyceride (ether) sulfates corresponding to formula **(I)**: 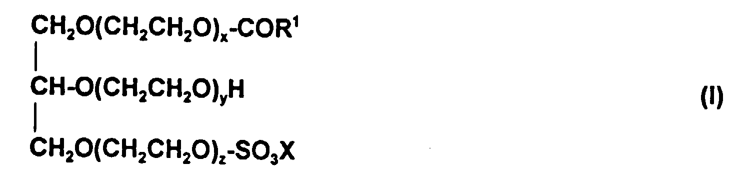 in which R¹CO is a linear or branched acyl group containing 6 to 22 carbon atoms, x, y and z together represent 0 or numbers of 1 to 30 and X is an alkali metal or alkaline earth metal,
are used.

3. The use claimed in claims 1 and 2, characterized in that fatty acid isethionates corresponding to formula **(II)**:
**R**^{**2**}**CO-CH**_{**2**}**CH**_{**2**}**SO**_{**3**}**X** (II)
in which R²CO is an aliphatic, linear or branched, saturated or unsaturated acyl group containing 6 to 22 carbon atoms and X is an alkali metal and/or alkaline earth metal, ammonium, alkylammonium, alkanolammonium or glucammonium,
are used.

4. The use claimed in claims 1 to 3, characterized in that fatty acid taurates corresponding to formula **(III)**: 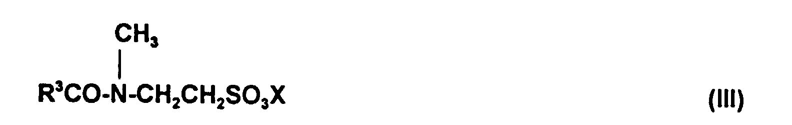 in which R³CO is an aliphatic, linear or branched or unsaturated acyl group containing 6 to 22 carbon atoms and X is an alkali metal and/or alkaline earth metal, ammonium, alkylammonium, alkanolammonium or glucammonium, are used.

5. The use claimed in claims 1 to 4, characterized in that fatty acid sarcosinates corresponding to formula **(IV)**: 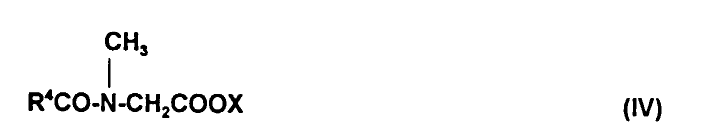 in which R⁴CO is an aliphatic, linear or branched or unsaturated acyl group containing 6 to 22 carbon atoms and X is an alkali metal and/or alkaline earth metal, ammonium, alkylammonium, alkanolammonium or glucammonium, are used.

## Revendications

1. Utilisation de mélanges détergents doux pour l'obtention de produits de lavage de vaisselle à la main, shampooings pour cheveux et bains moussants,
caractérisée en ce qu'
on utilise
(a) des (éther)sulfates de monoglycérides et ,
(b) des produits de condensation d'acides gras choisis dans le groupe formé par :
(b1) les iséthionates d'acides gras,
(b2) les taurates d'acides gras, et/ou
(b3) les sarcosinates d'acides gras,
dans un rapport pondéral des composants (a) et (b) de 50:50 à 80:20.

2. Utilisation selon la revendication 1,
caractérisée en ce qu'
on utilise des (éther)sulfates de monoglycérides de formule (I) 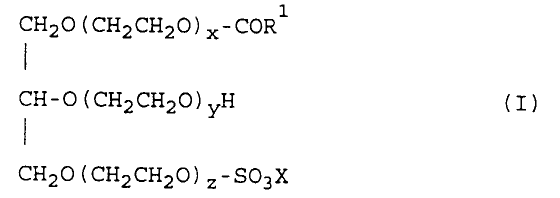 dans laquelle R¹CO représente un radical acyle linéaire ou ramifié ayant de 6 à 22 atomes de carbone, x, y, z valent au total 0 ou des nombres compris entre 1 et 30 et X représente un métal alcalin ou alcalino-terreux.

3. Utilisation selon les revendications 1 et 2,
caractérisée en ce qu'
on utilise les iséthionates d'acides gras de formule (II),
R²CO-CH₂CH₂SO₃X (II)
dans laquelle R²CO représente un radical acyle aliphatique, linéaire ou ramifié, saturé ou insaturé ayant de 6 à 22 atomes de carbone, et X représente un métal alcalin et/ou un métal alcalino-terreux, l'ammonium, un alkylammonium, un alcanolammonium ou glucammonium.

4. Utilisation selon les revendications 1 à 3,
caractérisée en ce qu'
on utilise des taurates d'acides gras de formule (III), 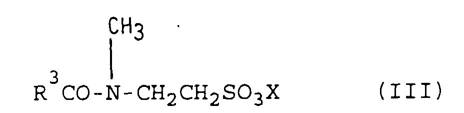 dans laquelle R³CO représente un radical acyle aliphatique, linéaire ou ramifié, saturé ou insaturé ayant de 6 à 22 atomes de carbone et X représente un métal alcalin et/ou alcalino-terreux, l'ammonium, un alkylammonium, alcanolammonium ou glucammonium.

5. Utilisation selon les revendications 1 à 4,
caractérisée en ce qu'
on utilise les sarcosinates d'acides gras de formule (IV) 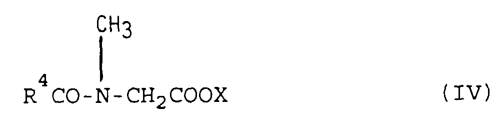 dans laquelle R⁴CO représente un radical acyle aliphatique, linéaire, ramifié ou insaturé ayant de 6 à 22 atomes de carbone et X représente un métal alcalin et/ou alcalino-terreux, l'ammonium, un alkylammonium, alcanolammonium ou glucammonium.
